Europäisches Patentamt

European Patent Office

Office européen des brevets

(19)

(11) Numéro de publication: **0 077 856 B1**

(12) # FASCICULE DE BREVET EUROPEEN

(45) Date de publication du fascicule du brevet:
21.05.86

(51) Int. Cl.⁴: **C 12 N 1/04, C 02 F 3/34**

(21) Numéro de dépôt: 81401708.3

(22) Date de dépôt: 27.10.81

(54) Procédé de préparation d'un inoculum pour l'entretien de fosses septiques et analogues, comportant des micro-organismes déshydratés et des enzymes, et inoculums préparés par ce procédé.

(43) Date de publication de la demande:
04.05.83 Bulletin 83/18

(45) Mention de la délivrance du brevet:
21.05.86 Bulletin 86/21

(84) Etats contractants désignés:
AT BE CH DE GB IT LI LU NL SE

(56) Documents cités:
DE - C - 283 882
FR - A - 1 103 676
FR - A - 1 268 165
FR - A - 2 147 522
FR - A - 2 226 364
FR - A - 2 482 130
US - A - 3 898 132

CHEMICAL ABSTRACTS, volume 82, no. 21, 26 mai 1975,
page 269, abrégé 135623u COLUMBUS OHIO (US)
PATENT ABSTRACTS OF JAPAN, volume 1, no. 84, 6
août 1977, page 1773C77

(73) Titulaire: **SOCIETE INDUSTRIELLE DES PRODUITS CHIMIQUES LUMER, 16-18, rue Paul Bert, F-93170 Bagnolet (FR)**

(72) Inventeur: **Lepretre, Maurice Résidence Dauphine, Pavillon Joffre 17 rue de la Croix Rouge, F-78430 Louveciennes (FR)**

(74) Mandataire: **CABINET BONNET-THIRION, 95 Boulevard Beaumarchais, F-75003 Paris (FR)**

## Description

L'invention a trait à un procédé de préparation d'un inoculum destiné au rééquilibrage biologique de fosses septiques, digesteurs d'épuration d'effluents et analogues, suivant lequel on ajoute à des cultures déshydratées de micro-organismes sélectionnés, des enzymes spécifiques de lyse de matières organiques à longues chaînes, et des charges d'excipient. L'invention se rapporte également à des inoculums ainsi prépares.

La dégradation biologique des matières organiques des effluents, telle qu'elle se produit dans les fosses septiques, digesteurs d'épuration d'effluents, est réalisée par l'action de micro-organismes, bactéries notamment, avec pour termes ultimes essentiels méthane, gaz carbonique et eau, accompagnés de boues minéralisées. En gros cette dégradation a lieu en deux temps, une lyse des matières organiques à longues chaînes sous l'action d'exo-enzymes ou co-enzymes secrétées par les micro-organismes dans le milieu aqueux, dit généralement substrat, cette lyse produisant des lysats formés de sucres principalement, puis consommation de lysats par les micro-organismes et rejet de produits ultimes de dégradation. Bien entendu les effluents contiennent pratiquement toujours une certaine proportion de composés organiques directement consommables par les micro-organismes. L'activité biologique des micro-organismes, et notamment leur reproduction multiplicative, est étroitement liée à la consommation des composés nutritifs, lysats et composés directement consommables, et à la concentration dans le substrat des produits ultimes de dégradation. En raison du court cycle de reproduction multiplicative des micro-organismes, et si le temps de contact du substrat avec les micro-organismes est suffisamment long, la population de micro-organismes, ou biomasse, s'adapte à la quantité de composés nutritifs présents dans le substrat; biomasse et substrat sont pratiquement en équilibre biologique.

Par ailleurs, les micro-organismes appartiennent à des espèces différentes qui consomment préférentiellement certains composés organiques. Certaines espèces rejettent des déchets qui sont consommables par d'autres, donnant lieu à des associations symbiotiques. L'équilibre biologique s'établit au sein des associations symbiotiques, et par concurrence des associations.

Toutefois il arrive que cet équilibre biologique se trouve détruit, soit que le flux d'apport de matières organiques, ou flux d'influents, subisse des variations trop brutales, soit que l'apport de matières dont la dégradation suppose une lyse préalable soit excessif devant la quantité d'exo-enzymes que la biomasse est en état de secréter (amidons, protéines, graisses-cellulose), soit encore que les influents contiennent des produits toxiques pour la biomasse (détergents et produits chlorés, tel qu'eau de javel) ou inhibiteurs de reproduction (antibiotiques). Il se produit alors, soit une accumulation de matières non dégradées, soit des déséquilibres d'associations symbiotiques svec dépérissement des espèces en aval des chaînes symbiotiques, dont les actions sont plus ou moins cumulatives. Les accumulations de matières non dégradées ont tendance à bloquer l'oxygénation du substrat, et les micro-organismes aérobies dépérissent. Par exemple, les composés contenant de l'azote, du soufre et du phosphore subissent des dégradations réductrices, avec formation d'amines, sulfures et phosphures.

Pour remédier à ces déséquilibres, qui se traduisent par un fonctionnement défectueux des fosses, on a, pendant longtemps, été obligé de procéder à des curages, pour eliminer les composés facteurs de deséquilibre. On a proposé également de réensemencer les fosses avec des inoculums, composés de cultures de micro-organismes sélectionnés, pour correspondre sensiblement à la flore bactérienne présente dans les fosses en bon équilibre de fonctionnement. Comme la stabilité de cultures, où les micro-organismes sont en activité, n'est possible que si le substrat de culture est en constant renouvellement, les inoculums doivent comporter des microorganismes mis en état de léthargie, où les échanges de nutrition et d'excrétion, ainsi que la reproduction, sont suspendus, sans que les micro-organismes soient tués pour autant, afin de reprendre leur activité au contact d'un milieu favorable. La plupart des micro-organismes sont en léthargie lorsque leurs échanges osmotiques avec le substrat ambiant sont bloqués, notamment par congélation ou deshydratation. Classiquement, pour obtenir des inoculums stables, on soumet les cultures de micro-organismes à une lyophilisation. Or la lyophilisation met en oeuvre un matériel complexe de capacité de traitement relativement faible, et par voie de conséquence constitue un stade opératoire dont le coût se répercute fâcheusement sur le coût global d'un produit destiné à une grande diffusion.

Par ailleurs, la conservation des cultures lyophilisées suppose qu'elles soient maintenues à l'abri de l'humidité de l'air; en conséquence, ces cultures lyophilisées sont généralement enfermées par doses dans des capsules, qui seront résorbées ultérieurement dans le substrat, à la façon de produits médicamenteux ingestibles.

Il est connu d'ajouter aux cultures de micro-organismes déshydratées par lyophilisation des enzymes spécifiques de la lyse des principaux constituants organiques à longues chaînes dont l'excès provoque des ruptures d'équilibre biologique dans les fosses septiques. Ces constituants à longue chaîne sont surtout des graisses, des matières protéiques des amidons et des celluloses, de sorte que les enzymes ajoutées sont des lipase, protéase, amylase et cellulase. L'action de ces enzymes ajoutées accélère la dégradation des matières organiques à longue chaîne, de façon à enrichir le substrat en lysats directement attaquables par les micro-organismes, qui peuvent ainsi proliférer rapidement et excréter ensuite une quantité de co-enzymes suffisante pour rétablir l'équilibre biologique.

Par ailleurs les composants actifs des inoculums, cultures déshydratées de micro-organismes et enzymes, doivent être apportés en quantités qui représentent des volumes et poids très faibles, dont le dosage est difficile. Aussi ajoute-t-on des excipients sous forme de charges pulvérulentes.

Par ailleurs le rétablissement rapide de l'équilibre biologique implique une dissémination rapide du produit dans le volume du substrat. A cet égard, la présentation du produit en capsules est défavorable, la dissémination ne pouvant se produire qu'après résorption de l'enveloppe, qui n'est obtenue pratiquement qu'après que l'agitation du substrat, consécutive à l'arrivée du flux d'eau d'entraînement de la capsule, ait cessé. En outre la dispersion est freinée fréquemment par la présence de matières non dégradées (graisses, amidons).

La présentation du produit en solution, favorable à la dispersion, est désavantageuse sous l'aspect de la conservation étant donné que les populations de micro-organismes, qui sont rendus actifs par la présence d'eau sans y trouver une composition nutritive convenable, régressent rapidement.

L'invention a pour objet un procédé de préparation d'inoculums pour le rééquilibrage biologique de fosses septiques et digesteurs, qui allient une grande activité spécifique à une bonne conservation.

L'invention a également pour objet un procédé de préparation d'inoculums peu onéreux en production de masse.

A ces effets l'invention propose un procédé de préparation d'un inoculum destiné au rééquilibrage biologique de fosses septiques et digesteurs d'épuration d'effluents, suivant lequel on ajoute à des cultures de micro-organismes sélectionnées, mises en léthargie par déshydratation, des enzymes spécifiques de lyse de matières organiques à longues chaînes, caractérisé en ce que l'on déshydrate lesdites cultures par addition d'un tamis moléculaire hydrophile constitué par une zéolithe, en rapport pondéral de 4 à 8 par rapport auxdites cultures, et en ce que, on ajoute à l'ensemble tamis molécalaire et cultures un dispersant non ionique pulvérulent.

On savait par les brevets japonais Nos. 76 116.282 et 5.247.484 que des cultures de micro-organismes pouvaient être conservées sans dégradation importante de la population, par un mélange avec des zéolithes déshydratées. Le premier brevet cité ci-dessus précise que le rapport pondéral zéolithe culture est compris entre 10 et 50. Les travaux qui ont conduit à la présente invention ont permis de découvrir que, au-delà de la conservation des cultures, les inoculums comprenant des cultures en léthargie par déshydration, des enzymes spécifiques de lyse de molécules à longue chaîne et des charges éventuelles d'excipients, étaient susceptibles de garder toute leur efficacité, voire leur population de micro-organismes progresser si des tamis moléculaires hydrophiles étaient utilisés à saturation pour déshydrater les cultures, ce que correspond pour une zéolithe à un rapport pondéral de 4 à 8 par rapport aux cultures, et si on ajoutait un dispersant non ionique pulvérulent. On peut supposer que le mélange cultures de micro-organisme-zéolith hydrophile constitue un milieu sensiblement neutre, les cultures ne pouvant être mises en contact avec les autres composants de l'inoculum par adsorption conjointe par la zéolithe. En outre l'utilisation d'un dispersant non ionique pulvérulent évite l'apport d'eau au mélange, ce qui risquerait de réhydrater les cultures. Et l'on remarquera que le processus de préparation de l'inculum est aisé à mettre en oeuvre à l'échelle industrielle.

De préférence, on ajoute à l'ensemble tamis moléculaire et cultures, outre le dispersant non ionique, des charges d'excipients anhydres.

On choisit les souches origines des cultures de préférence parmi des espèces normalement présentes dans les fosses septiques ou les digesteurs, à savoir Enterobacter Cloacae, Pseudomonas Fluorescens, Serratia Marcescens, Bacillus Cereus et Bacillus Subtilis, que l'on abrégera fréquemment ci-après respectivement par E.C., P.F., S.M., B.C., et B.S.  De préférence également les enzymes seront choisies, en fonction des matières organiques difficilement dégradables présentes, parmi la protéase, la lipase, l'amylase et la cellulase.

En procédé préféré on composera extemporanément un mélange de cultures provenant de souches distinctes, on dispersers par agitation le tamis moléculaire dans le mélange, et on ajoutera successivement les enzymes, le dispersant non ionique et enfin les charges d'excipients.

Les cultures sont préparées, en disposition préférée, par ensemencement avec la souche correspondante d'un substrat comprenant, par litre, 4 g de glucose, 3 g d'extrait de levure, 3 g d'extrait de viande de boeuf, 5 g de chlorure de sodium et 2 g d'amidon, dissous dans une eau de source peu mineralisée, puis incubation vers 37°C jusqu'à développement convenable de la culture, contrôlé par numération de la population. On obtient ainsi des cultures bien reproductibles.

Les caractéristiques et avantages de l'invention ressortiront d'ailleurs de la description qui va suivre, assortie d'exemples.

Au depart, il convenait de revoir le problème du rééquilibrage biologique de fosses septiques et digesteurs d'effluents dans tous ses aspects, c'est-à-dire les conditions de fonctionnement en équilibre, les manifestations et causes des déséquilibres, et les insuffisances des solutions apportées dans l'état de la technique. Ces aspects ont été évoqués plus haut.

Il est alors apparu que les perfectionnements devaient porter essentiellement sur deux points : l'ensemencement des fosses septiques et digesteurs avec des cultures de microorganismes specifiques dans un état d'activité efficace et contrôlé, et l'etablissement de conditions favorables à la multiplication des micro-organismes dans les moments qui suivent l'ensemencement.

Sur le premier point, il était clair que l'ensemencement devait être réalisé avec des cultures sélectionnées, à un stade précédent la degénérescence. La technique connue consistant à fixer l'état de croissance de la culture par la congélation, suivie d'une lyophilisation pour permettre le maintien en léthargie de la culture et sa conservation à l'état de croissance fixé, technique qui a été développée pour les applications biomédicales et pharmaceutiques des micro-organismes, apparaissait comme peu compatible avec une production industrielle, tant par son prix de revient que par les difficultés de manipulation et de dosage des cultures lyophilisées en raison du faible volume propre des microorganismes. Par ailleurs la lyophilisation étant très bien adaptée aux impératifs propres des applications pharmaceutiques et biomédicales, champ privilégié d'utilisation des micro-organismes à effets dosés, l'état de la technique n'offrait pas de processus de remplacement à la lyophilisation pour la mise en léthargie des micro-organismes.

Il était connu de séparer les constituants d'une solution selon la taille de leurs molécules au moyen de corps adsorbants poreux dits tamis moléculaires, dont le diamètre et les fonctions chimiques superficielles des pores permettent une adsorption sélective de molécules, notamment polaires, dont le diamètre est inférieur au diamètre des pores. On appellera hydrophiles les tamis moléculaires présentant une sélectivité pour l'adsorption de molécules polaires de taille comparable à celle des molécules d'eau. Une classe particulière de tamis moléculaires est constituée par des zéolithes, ou silicates hydratés.

Le déposant a découvert que les propriétés d'adsorption d'eau des tamis moléculaires pouvaient être utilisés avec succès à la déshydratation de micro-organismes, que les micro-organismes ainsi mis en léthargie reprenaient leur activité normale au contact d'un substrat nutritif convenable, et que la présence du tamis moléculaire n'avait alors pas d'effets secondaires fâcheux, et tout au contraire facilitait la dispersion dosée des micro-organismes en agissant comme diluant. Comme tamis moléculaire convenant bien à l'application considérée, on peut citer la zéolithe artificielle commercialisée par CECA sous le nom de "Siliporite NK 10"®.

Par ailleurs il a été constaté que la déshydratation s'effectuait dans les meilleures conditions lorsque la capacité d'adsorption d'eau de la quantité de tamis moléculaire correspondait sensiblement à la quantité d'eau dans la culture utilisée. Compte tenu de ce que, dans la culture, le volume propre des micro-organismes est négligeable devant le volume d'eau, et que la capacité d'adsorption du tamis moléculaire se situe dans une fourchette de rapport pondéral tamis moléculaire/eau de 4 à 8 (sensiblement 5 pour la zéolithe precitée), le processus de déshydratation typique comprend l'addition, dans un mélangeur à grande vitesse, à une masse déterminée de cultures, de 4 à 8 fois cette masse de tamis moléculaire et agitation jusqu'à siccité apparente complète.

Le choix des souches de micro-organismes a été guidé par les considérations suivantes. Les espèces cultivées doivent correspondre aux espèces prédominantes dans les fosses septiques ou digesteurs, et les souches doivent être aisément diaponibles et suffisamment stables pour que lea cultures soient reproductibles; enfin il est souhaitable de choisir des souches appartenant à des sous-espèces non pathogènea.

Les souches retenues par le déposant sont, dans la classification de l'Institut Pasteur - Paris

(E.C.) Enterobacter Cloacae 60.22
(B.C.) Bacillus Cereua A.30 CIP
(P.F.) Pseudomonas Fluorescens 56.90
(S.M.) Serratia Marcescens 53.101
(B.S.) Bacillus Subtilis 53.159

On soulignera que si certaines sous-espèces de Bacillua Subtilis sont considérées comme pathogènes, il existe un certain nombre de souches correspondant à des sous-espèces non pathogènes, et que la souche retenue mentionnée ci-dessus est particulièrement sûre à cet égard.

## EXEMPLE 1

Processus de culture.

Le processus comprend la préparation du milieu de culture, l'ensemencement et l'incubation, et le contrôle de la densité de population. Chaque souche doit être cultivée séparément. La composition du milieu de culture et les conditions d'incubation sont, par contre, pratiquement les mêmes.

A) Milieu de culture.

A environ 3/4 de litre d'une eau de source peu minéralisée (type Eau d'Evian) on ajoute 4 g de glucose, 3 g d'extrait de levure (MERCK), 3 g d'extrait de viande de boeuf (MERCK), 5 g de chloture de sodium et 2 g d'amidon; après dissolution des ajouts, on complète à un litre avec la même eau de source.

B) Ensemencement.

On place dans un incubateur à température réglée à 37° ± 0,5°C un récipient stérile contenant un volume connu de milieu de culture. Lorsque le milieu de culture a atteint la température de l'incubateur, on introduit dans le récipient un prélèvement de la souche voulue. Celle-ci a été repiquée, à partir du lyophilisat reçu, sur gélose dans des conditions classiques, en conformité avec les instructions générales de l'organisme fournisseur de souches.

C) Incubation.

La température de l'incubateur étant maintenue à 37° ± 0,5°C, on contrôle périodiquement l'état de la culture par numération, comme il sera précisé à l'alinéa suivant. Après une période de latence, d'environ 2 heures, au cours de laquelle la population ne subit pas de croissance appreciable, on observe une période de croissance d'allure exponentielle, d'environ 8 heures, puis un ralentissement progressif du taux de croissance. Après une incubation de 36 heures environ, la culture aura subi son plein développement, puis entamera une phase de régression. On arrête donc la culture un peu avant son plein développement.

D) Contrôle de la densite de population.

On a déterminé expérimentalement les densités de population correspondant à l'époque optimale d'arrêt. Celles-ci, exprimées en milliards d'individus par gramme de milieu de culture, ont été fixées à :

6 pour E.C. Enterobactet Cloacae;
5 pour P.F. Pseudomonas Fluorescens;
5 pour S.M. Serratia Marcescens;
4,7 pour B.C. Bacillus Cereus;
2 pour B.S. Bacillus Subtilis.

Pour contrôler la densité de population, on opère d'abord par dilutions successives d'un prélévement de la culture, pour arrivet à une densite estimée de quelques centaines d'individus au millilitre. On effectue ensuite une numération par la méthode "Millipore", avec absorption de 1 ml de culture diluée dans un filtre, et comptage des colonies qui se développent à partir de chaque individu à la surface du filtre.

L'arrêt des cultures est effectué par le processus suivant :

On effectue le mélange extemporané de quantités déterminées de cultures choisies, suivant l'application visée, pout correspondre sensiblement aux répartitions de population dans un digesteur en équilibre biologique, et ce mélange est placé dans un mélangeur à grande vitesse, type Nauta, et l'on ajoute un poids de Siliporite NK 10 égal à 5 fois le poids du mélange. On laisse toutnet jusqu'à siccité complète apparente.

**EXEMPLE 2**

Cultures déshydratées pour inoculum de fosse septique domestique.

Le mélange de cultures comprend 4,4 g de culture d'E.C. 1,4 g de B.C., 7,4 g de B.S., 3,4 g de P.F. et 3,4 g de S.M. On ajoute 100 g de Silipotire NK 10 ®.

**EXEMPLE 3**

Cultures déshydratées pout inoculum de digesteurs.

Le mélange comprend 15,7 g de culture d'E.C., 12,15 g de P.F. et 12,15 g de S.M. On ajoute deux cents grammes de Siliporite NK 10 ®.

Dès la fin de la déshydratation des cultutes, on introduit des enzymes spécifiques de lyse de matières organiques à longues chaînes, choisies pour correspondre en spécificités et quantités aux matiètes organiques présentes dans les influents de fosses ou digesteurs, et dont l'accumulation est cause de déséquilibre biologique. Ces enzymes sont essentiellement la protéase (500 ST), pour la lyse des protéines, l'amylase (THC 250), pour l'hydrolyse des amidons, la lipase (80.000 ST), pour l'hydrolyse des graisses, et la cellulose (50.000 ST) pour l'hydrolyse de la cellulose des papiers et résidus alimentaires.

Après l'introduction des enzymes, on ajoute un dispersant non ionique constitué par un condensat d'alcool stéaroylique avec un polyoxyde d'éthylène à 50 motifs environ, c'est-à-dire resultant de la polymériaation d'environ 50 molécules d'oxyde d'ethylène. Ce dispersant est commarcialisé par Witco Chemical sous le nom de Cetalox AT®.

Après cela on ajoute des charges d'excipients, comprenant du bicarbonate de sodium, tamponnant, et du chlorure de sodium. On précisera plus loin les compositions préférées d'inoculum. Dans l'ensemble ces charges auront pour rôle d'assurer, au cours de la dispersion dans le substrat à traiter, que la composition du substrat au contact des microorganismes est favorable à laur reprise d'activité, et éventuellemant de coopérer avec le dispersant pour assurer une dispersion rapide et efficace. En outre ces charges permettent da définir aisément des doses de cultures et d'enzymes.

**EXEMPLE 4**

Composition d'inoculum pour fosses septiques domestiques.

A la culture déshydratée de l'exemple 2 (120 g) on ajoute 1,200 kg de protéase, 0,120 kg d'amylase, 0,004 kg de lipase et 0,060 kg de cellulase, puis 2 kg de "Cetalox AT" ®, 5 kg de bicarbonate de sodium, 10 kg de talc, et 81,5 kg d'un mélange en parties égales de mica en poudre micronisée et de chlorure de sodium exempt d'iode. On obtient ainsi 100 kg d'inoculum en poudre.

Pour l'entretien normal d'une fosse septique classique, correspondant à l'utilisation par 3 personnes, on déposera chaque semaine 25 g environ de cet inoculum, et on l'entraînera dans la fosse par une chasse d'eau.

**EXEMPLE 5**

Composition d'inoculum pour digesteurs d'effluents.

A la culture déshydratée de l'exemple 3 (240 g) on ajoute 0,090 kg de lipase (80.000 ST), puis 6 kg de "Cetalox AT"®, et après 30 minutes, 0,500 kg de bicarbonate de sodium, 14 kg de phosphate disodique (anhydre), 14 kg de phosphate monosodique, et 15,17 kg de chlorure de sodium, pour obtenir 50 kg d'inoculum.

On remarquera que cet inoculum est destiné à des applications où les graisses constituent l'agent majeur d'engorgement. Par ailleurs les phosphates favorisent la réactivation des micro-organismes.

Cet inoculum trouve son utilisation dans les bassins de décantation municipaux, l'entretien des drains, boîtes à graisse, broyeurs d'immondices, éviers, puisards, l'entretien des porcheries (traitement des lisiers de porcs) dans les abattoirs...

Hors les traitements de chocs, pour la remise en état d'installations très déséquilibrées biologiquement, où l'on pourra utiliser des doses relativement élevées pour une action massive de la lipase, on comptera pour l'entretien normal environ 1 g par $m^3$ tous les 20 jours.

On remarquera que les charges d'excipients n'ont de rôle que lors des opérations d'inoculation du substrat, pour faciliter la détermination des doses, et éventuellement corriger localement la composition du substrat. Il en résulte que le produit actif, constitué par les cultures déshydratées par le tamis moléculaire, les enzymes et le dispersant pulvérulent, pourra être préparé et conservé sans charges d'excipients. Lors d'un traitement de choc nécessitant des doses élevées de produit actif, la présence de charges d'excipients peut s'svérer au moins inutile, sinon nuisible, étant donné que le dosage sur le produit actif ne présente plus de difficultés tandis que les charges pourraient former des boues dans le substrat, ou apporter des concentrations excessives en sel solubles. On préférera alors utiliser le produit actif pur, tel qu'il se présente dans les exemples 4 et 5 avant l'ajout de bicarbonate de sodium. Par ailleurs, comme l'ajout des charges d'excipients ne présente pas de difficultés particulières, on pourra, pour éviter un transport à grande distance de produits pondéreux, expédier le produit actif pur dans un pays lointain où sera effectué l'ajout des charges d'excipients avant la commercialisation.

Pour le contrôle de l'efficacité des inoculums le déposant a défini un substrat type, afin de déterminer la vitesse de digestion des différents composants et l'évolution des populations de micro-organismes.

Ce substrat a la composition suivante, en poids:

Eau 93,4
Saindoux 0,25
Savon 0,10 %
Amidon de blé 3,45 %
Cellulose 0,10
Gélatine 2,70

Le mélange est brassé vigoureusement, puis laisse au repos 24 heures. La viscosité, déterminée avec un viacomètre DRAG équipé d'un mobile A, et prise à trois vitesses de rotation $V_1$, $V_2$ et $V_3$ s'établit à:

$V_1$ 13,549 Pa.s
$V_2$ 6,116 Pa.s
$V_3$ 2,387 Pa.s

On notera la diminution de viscosité avec l'accroissement de vitesse de rotation du mobile de mesure.

En ajoutant au substrat, maintenu à une température moyenne d'environ 15°C 0,1 % en poids de l'inoculum selon l'exemple 3, la viscosité après 24 heures tombe à 5,4 $10^{-3}$ Pa.s. On rappelle que la viscosité de l'eau pure à 20°C est sensiblement de $10^{-3}$ Pa.s.

Bien entendu, l'invention n'est pas limitée aux exemples décrits, mais en embrasse toutes les variantes d'exécution. Notamment les quantités d'enzymes ajoutées aux cultures peuvent être ajustées, pour des applications particulières, proportionnellement aux teneurs en matières organiques à longues chaînes des effluents, les charges d'excipients peuvent être adaptées pour compenser des carences de ces effluents en azote ou en phosphore.

**Revendications**

1. Procédé de préparation d'un inoculum destiné au rééquilibrage biologique de fosses septiques et digesteurs d'épuration d'effluents, suivant lequel on ajoute à des cultures de microorganismes sélectionnées, mises en léthargie par déshydratation, des enzymes spécifiques de lyse de matières organiques à longues chaînes, caractérisé en ce que l'on déshydrate les dites cultures par addition d'un tamis moléculaire hydrophile constitué par une zéolithe, en rapport pondéral de 4 à 8 par rapport auxdites cultures, et en ce que l'on ajoute à l'ensemble tamis moléculaire et cultures un dispersant non ionique pulvérulent.

2. Procédé suivant la revendication 1, caractérisé en ce que l'on ajoute à l'ensemble tamis moléculaire et cultures, outre le dispersant non ionique des charges d'excipient anhydre.

3. Procédé suivant la revendication 1 ou la revendication 2, caractérisé en ce que le dispersant non ionique est un condensat d'alcool stéaroylique avec un polyoxyde d'éthylène à 50 motifs environ.

4. Procédé suivant la revendication 1 ou la revendication 2, caractérisé en ce que lesdites cultures proviennent de souches de micro-organismes choisis dans le groupe comprenant Enterobacter Cloacae (E.C.), Pseudomonas Fluorescens (P.F.), Serratia Marcescens (S.M.), Bacillus Cereus (B.C.) et Bacillus Subtilis (B.S.).

5. Procédé suivant la revendication 1 ou la revendication 2, caractérisé en ce que lesdites enzymes sont choisies dans le groupe comprenant la protéase, l'amylase, la lipase et la cellulase.

6. Procédé suivant la revendication 2, caractérisé en ce que l'on compose extemporanément un mélange de cultures de souches de micro-organismes distincts, on disperse par agitation le tamis moléculaire dans le mélange de culture, puis on ajoute successivement les enzymes, le dispersant non ionique pulvérulent et enfin les charges d'excipients.

7. Procédé suivant la revendication 6, incluant la préparation desdites cultures, caractérisé en ce que l'on ensemence avec la souche de micro-organismes distincts un substrat comprenant, par litre, 4 g de glucose, 3 g d'extrait de levure, 3g d'extrait de viande de boeuf, 5 g de chlorure de sodium et 2 g d'amidon, dissous dans la quantité nécessaire d'eau de source peu minéralisée, et on incube le substrat ensemencé à une température de 37° ● 0,5°C pendant 12 à 36 heures.

8. Procédé suivant la revendication 7, caractérisé en ce que l'on termine l'incubation lorsque la densité de population de micro-organismes dans le substrat atteint une valeur choisie.

9. Procédé suivant les revendications conjointes 4 et 7, caractérisé en ce que la valeur choisie de densité de population, exprimée en milliards d'individus par gramme de substrat, est d'environ 6 pour E.C., 5 pour P.F. et S.M., 4,7 pour B.C. et 2 pour B.S.

10. Inoculum pour fosses septiques domestiques préparé par procédé suivant la revendication 9, caractérisé en ce qu'il comprend 20 g de mélange de cultures composé de 4,4 g de E.C., 1,4 g de B.C., 7,4 g de B.S., 3,4 g de P.F. et 3,4 g de S.M., 100 g de tamis moléculaire zéolithe, 1,2 kg de protéase, 0,12 kg d'amylase, 0,004 kg de lipase et 0,060 kg de cellulase, 2 kg de condensat d'alcool stéaroylique avec un polyoxyéthylène à 50 motifs environ, 5 kg de bicarbonate de sodium, 10 kg de talc et 81,5 kg d'un mélange en parties égales de mica en poudre micronisée et de chlorure de sodium exempt d'iode.

11. Inoculum pour l'entretien de digesteurs d'épuration d'effluents, préparé par procédé suivant la revendication 8, caractérisé en ce qu'il comprend 40 g de mélange de cultures composé de 15,7 g de E.C., 12,15 g de P.F. et 12,15 g de S.M., 200 g de tamis moléculaire zéolithe, 90 g de lipase, 6 kg de condensat d'alcool stéaroylique avec un polyoxyéthylène à 50 motifs environ, 0,5 kg de bicarbonate de sodium, 14 kg de phosphate disodique, 14 kg de phosphate monosodique et 15,17 kg chlorure de sodium exempt d'iode.

**Patentansprüche**

1. Verfahren zur Herstellung eines Inokulums für die Wiederherstellung des biologischen Gleichgewichts von Klärgruben und Behältern zur Reinigung von Abwässern, bei dem man Kulturen von ausgewählten Mikroorganismen, die durch Dehydratisierung inaktiviert sind, spezifische Enzyme für den Abbau von organischen Materialien mit langen Ketten zusetzt, dadurch gekennzeichnet, daß man die genannten Kulturen durch Zugabe eines hydrophilen, aus einem Zeolithen aufgebauten Molekularsiebes mit einem Gewichtsverhältnis von 4 bis 8, bezogen auf das Gewicht dieser Kulturen, dehydratisiert, und daß man zu den mit dem Molekularsieb versehenen Kulturen ein nicht-ionisches, feinpulverisiertes Dispersionsmittel zugibt.

2. Verfahren nach Anspruch 1, dadurch gekennzeichnet, daß man zu den mit dem Molekularsieb versehenen Kulturen außer dem nicht-ionischen Dispersionsmittel gewisse Mengen an wasserfreien Hilfsstoffen gibt.

3. Verfahren nach Anspruch 1 oder 2, dadurch gekennzeichnet, daß das nicht-ionische Dispersionsmittel ein Kondensat von Stearylalkohol mit einem Polyethylenoxid mit ungefähr 50 Einheiten ist.

4. Verfahren nach Anspruch 1 oder 2, dadurch gekennzeichnet, daß die genannten Kulturen von Stämmen von Mikroorganismen, ausgewählt aus der Gruppe Enterobacter Cloacae (E.C.), Pseudomonas Fluorescens (P.F.), Serratia Marcescens (S.M.), Bacillus Cereus (B.C.) und Bacillus Subtilis (B.S.), herrühren.

5. Verfahren nach Anspruch 1 oder 2, dadurch gekennzeichnet, daß die genannten Enzyme aus der Gruppe Protease, Amylase, Lipase und Cellulase ausgewählt sind.

6. Verfahren nach Anspruch 2, dadurch gekennzeichnet, daß man eine Mischung der Kulturen von Stämmen unterschiedlicher Mikroorganismen frisch zusammenstellt, unter Rühren das Molekularsieb in der Kulturmischung dispergiert, dann nacheinander die Enzyme, das nicht-ionische, feinpulverisierte Dispersionsmittel und schließlich die gewissen Mengen an Hilfsstoffen zusetzt.

7. Verfahren nach Anspruch 6, einschließlich der Herstellung der genannten Kulturen, dadurch gekennzeichnet, daß man mit dem Stamm der unterschiedlichen Mikroorganismen ein Substrat impft, das je Liter 4 g Glucose, 3 g Hefeextrakt, 3 g Rindfleischextrakt, 5 g Natriumchlorid und 2 g Stärke enthält, aufgelöst in der notwendigen Menge Wasser einer Quelle mit geringem Mineralgehalt, und man das geimpfte Substrat bei einer Temperatur von 37° ± 0,5°C 12 bis 36 Stunden lang inkubiert.

8. Verfahren nach Anspruch 7, dadurch gekennzeichnet, daß man die Inkubation beendet, wenn die Populationsdichte der Mikroorganismen in dem Substrat den gewünschten Wert erreicht hat.

9. Verfahren nach den beiden Ansprüchen 4 und 7, dadurch gekennzeichnet, daß der gewünschte Wert der Populationsdichte, ausgedrückt in Milliarden der Individuen je Gramm Substrat, ungefähr 6 für E.C., 5 für P.F. und S.M., 4,7 für B.C. und 2 für B.S. beträgt.

10. Inokulum für Haushalt-Klärgruben hergestellt nach dem Verfahren nach Anspruch 9, dadurch gekennzeichnet, daß es 20 g der Mischung der Kulturen, zusammengesetzt aus 4,4 g E.C., 1,4 g B.C., 7,4 g B.S., 3,4 g P.F. und 3,4 g S.M., 100 g Zeolith-Molekularsieb, 1,2 kg Protease, 0,12 kg Amylase, 0,004 kg Lipase und 0,060 kg Cellulase, 2 kg Kondensat von Stearylalkohol mit einem Polyethylenoxid mit ungefähr 50 Einheiten, 5 kg Natriumbicarbonat, 10 kg Talkum und 81,5 kg einer Mischung aus gleichen Teilen von mikronisiertem Glimmerpulver und von jodfreiem Natriumchlorid enthält.

11. Inokulum für die Instandhaltung von Behältern zur Reinigung von Abwässern, hergestellt nach dem Verfahren nach Anspruch 8, dadurch gekennzeichnet, daß es 40 g der Mischung der Kulturen, zusammengesetzt aus 15,7 g E.C., 12,15 g P.F. und 12,15 g S.M., 200 g Zeolith-Molekularsieb, 90 g Lipase, 6 kg Kondensat von Stearylalkohol mit einem Polyethylenoxid mit ungefähr 50 Einheiten, 0,5 kg Natriumbicarbonat, 14 kg Dinatriumphosphat, 14 kg Mononatriumphosphat und 15,17 kg jodfreies Natriumchlorid enthält.

## Claims

1. A process for preparing an inoculum intended for restoring the biological balance of septic tanks and effluent purification digesters, which comprises adding to selected micro-organism cultures which are put into a state of lethargy by dehydration, specitic enzymes for lysis of longchain organic materials, characterised by dehydrating said cultures by the addition of a hydrophilic molecular sieve formed by a zeolite, in a ratio by weight of from 4 to 8 with respect to said cultures, and adding to the assembly of the molecular sieve and the cultures a powdery non-ionic dispersant.

2. A process according to claim 1 characterised in that besides the non-ionic dispersant, anhydrous excipient fillers are added to the assembly of the molecular sieve and the cultures.

3. A process according to claim 1 or claim 2 characterised in that the non-ionic dispersant is a condensate of stearoyl alcohol with a polyethylene oxide having about 50 units.

4. A process according to claim 1 or claim 2 characterised in that said cultures originate from strains of micro-organisms selected from the group comprising Enterobacter Cloacae (E.C.), Pseudomonas Fluorescens (P.F.), Serratia Marcescens (S.M.), Pacillus Cereus (B.C.) and Bacillus Subtilis (B.S.).

5. A process according to claim 1 or claim 2 characterised in that said enzymes are selected from the group comprising protease, amylase, lipase and cellulase.

6. A process according to claim 2 characterised by extemporaneously composing a mixture of cultures of strains of separate micro-organisms, dispersing the molecular sieve in the culture mixture by agitation, and then successively adding the enzymes, the powder non-ionic dispersant and finally the excipient fillers.

7. A process according to claim 6 including the preparation of said cultures characterised by seeding with the strain of separate microorganisms a substrate comprising, per litre, 4 g of glucose, 3 g of yeast extract, 3 g of beef extract, 5 g of sodium chloride and 2 g of starch, dissolved in the necessary amount of non-mineralised spring water, and incubating the seeded substrate at a temperature of 37° ± 0.5°C for a period of 12 to 36 hours.

8. A process according to claim 7 characterised in that incubation is terminated when the micro-organism population density in the substrate reaches a selected value.

9. A process according to claims 4 and 7 in combination characterised in that the selected value in respect of population density, expressed in terms of thousands of millions of individuals per gram of substrate, is approximately 6 for E.C., 5 for P.F. and S.M., 4.7 for B.C. and 2 for B.S.

10. An inoculum for domestic septic tanks prepared by the process according to claim 9 characterised in that it comprises 20 g of mixture of cultures made up of 4.4 g of E.C., 1.4 g of B.C., 7.4 g of B.S. 3 4 g of P.F. and 3.4 g of S.M., 100 g of zeolite molecular sieve, 1.2 kg of protease, 0.12 kg of amylase, 0.004 kg of lipase and 0.060 kg of cellulase, 2 kg of condensate of stearoyl alcohol with a polyoxyethylene having about 50 units, 5 kg of sodium bicarbonate, 10 kg of talc and 81.5 kg of a mixture in equal parts of mica in the form of micronized powder and sodium chloride which is free from iodine.

11. Inoculum for the maintenance of effluent purification digesters, prepared by a process according to claim 8, characterised in that it comprises 40 g of a mixture of cultures made up of 15.7 g of E.C., 12.15 g of P.F. and 12.15 g of S.M., 200 g of zeolite molecular sieve, 90 g of lipase, 6 g of condensate of stearoyl alcohol with a polyoxyethylene having about 50 units, 0.5 kg of sodium bicarbonate, 14 kg of disodium phosphate, 14 kg of monosodium phosphate and 15.17 kg of sodium chloride which is free from iodine.